# EUROPEAN PATENT APPLICATION

(11) **EP 4 397 345 A1**
(43) Date of publication of application: **10.07.2024**
(21) Application number: 22863740.1
(22) Date of filing: 29.08.2022
(51) Int. Cl.: A61M 16/00

(54) **SELF-CONTAINED EMERGENCY SYSTEM AND METHOD FOR USE IN THE EVENT OF RESPIRATORY ARREST, FOR CARDIOPULMONARY RESUSCITATION, USING A VENTILATION MASK WITH A CO2 SENSOR, A SELF-REGULATING OXYGEN SOURCE AND A CERVICAL SUPPORT**

(30) Priority: 30.08.2021 CO 21011484
(71) Applicant: Montejo Tarazona, Javier, Neiva (Huila) (CO)
(72) Inventor: Montejo Tarazona, Javier, Neiva (Huila) (CO)
(74) Representative: Morbidini, Marco
(86) International application number: PCT/IB2022/058072
(87) International publication number: WO 2023/031765

(57) **Abstract**

The present invention relates to a self-contained emergency system and method for use in the event of respiratory arrest, for cardiopulmonary resuscitation, using a ventilation mask with a CO2 sensor, a self-regulating oxygen source and a cervical support, characterised in that it can be operated by persons with no emergency medical training, due to the fact that it is self-contained, and the supply of oxygen, in terms of volume and frequency, is calculated using software, based on the patient's age, and in turn, the system allows the patient's airways to be expanded by means of the cervical support, enabling an improved, efficient supply of oxygen in the event of respiratory arrest. This invention is characterised in that the ventilation mask has a CO2 venting valve and a CO2 sensor connected to software that determines the respiratory status of the patient. Likewise, the invention comprises a method, established in steps, for performing the respiratory arrest resuscitation action on patients using the system described herein. The field of application of the present invention is directed toward the healthcare sector, its purpose being emergency pulmonary resuscitation in patients with respiratory arrest.

## Description

This invention relates to an autonomous emergency method and system for respiratory failure and pulmonary reanimation via a ventilation mask equipped with a CO2 sensor, auto-regulated oxygen source, and cervical support. The application for the system corresponds to the medical and health field and has the goal of emergency reanimation in respiratory failure. The method and system can be operated by people with no medical emergency training, due to the system's autonomy in providing the correct volume and frequency of oxygenation. This autonomy is determined by software embedded in the system, which is set by entering the patient's age. Additionally, the system's cervical support allows for the patient's airway management to be adequate. This invention is further characterized by a ventilation mask that has both a CO2 vent and a CO2 sensor connected to software that determines the patient's respiratory state. As a last component, the invention has a step-by-step method that will allow the person providing the resuscitation to follow coherently.

### TECHNOLOGICAL SECTOR:

This invention is located within the biomedical, electronic, and software engineering sectors.

### BACKGROUND OF THE INVENTION:

This invention emerged from noticing the high degree of expertise and training required for people with no medical background to provide emergency assistance to respiratory failure patients. Current systems operate with a high degree of difficulty and their misuse has resulted in death or patient side-effects due to late reanimation. These issues prompted the inventor to create a system that addressed the need for an emergency system that could be safe, efficient in attending to emergency respiratory failure, as well as easy to use by someone with no training. The problems that occurred with previous systems were solved by giving the autonomous emergency method and system autonomy and predictability in its design, as well as in its components. To support the requirements of this novel invention, as well as differentiate it from other systems it was important to perform technological monitoring that showed current patents and their state of the art. The following patents were considered:

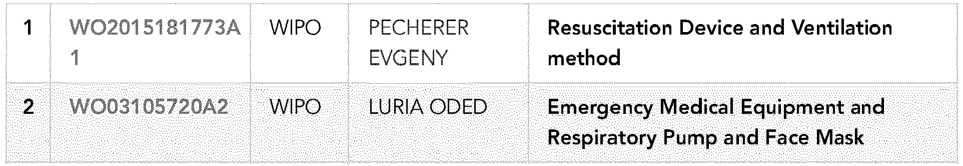

Prima facie evidence shows a common denominator in the state of the art of existing patents: none of the current inventions or featured patents monitored during the technological phase present a system and method in respiratory failure assistance that sets forward a reanimation and oxygenation action specific to the personal qualities of the user or patient -regardless of age- nor do they provide autonomy of the system itself, and ease of use by untrained assistance providers.

Such statements result when analyzing the differences between the Autonomous Emergency System for Respiratory Failure and the current patents found in the state of the art. Such is the case of the patent WO20151 81773A1 , which is presented as a device comprised of a mixing chamber, a compressor that can operate at two or more levels of potency, and a controllable pressure respirator mask. The differences between the two systems are that AIR does not generate a mixing of gases, and only provides oxygen; additionally, the pressure is given by a tank containing liquefied oxygen, and its release is controlled by electric valves that dose quantities according to regulated protocols. In contrast, WO2015181773A1, has a compressor and a controllable pressure respirator that controls the pressure of the provided mix, which makes it extremely heavy and difficult to transport. The Autonomous Emergency System for Respiratory Failure does not need such elements to execute its function, as will be shown in the detailed description of the invention.

Claim 47 further shows that WO2015181773A1 has been designed exclusively for neonatal patients, clearly separating its function from that of the Autonomous Emergency System for Respiratory Failure, which has been designed for adults and children.

Additionally, international patent WO03105720A2 shows a difference in the following: it implements a respiratory pump that is connected to the pressurized oxygen tank, which sets the system in action. The pump can also be connected to the mask to provide oxygenation and discharging exhalations into the environment, that is to say, supply and dosage are managed through the pump and its components. This system also contains a detachable headrest designed to hold the patient's neck if they need medical treatment -or to facilitate the placement of the face mask. The Autonomous Emergency System for Respiratory Failure includes a cervical support that has been designed to provide successful airway management through its positioning, which is integrated into the mask itself.

The Autonomous Emergency System for Respiratory Failure is also equipped with two valves located inside the mask: a maximum positive pressure valve, as well as a maximum negative pressure release valve. Both of these avoid the pressure within the mask exceeding the positive and negative predetermined limits. The Autonomous Emergency System for Respiratory Failure also has a valve in the ventilation mask, that has the function of liberating the gases emitted by the patient in the exhalation process.

The reasons stated above, as well as the elements that will be further presented in the detailed description of the Autonomous Emergency System for Respiratory Failure, solidify the pertinence of granting a patent to the invention presented in this document as it complies with the requirements of novelty and inventive level, which its industrial applications and uses will support.

### DESCRIPTION OF THE FIGURES.

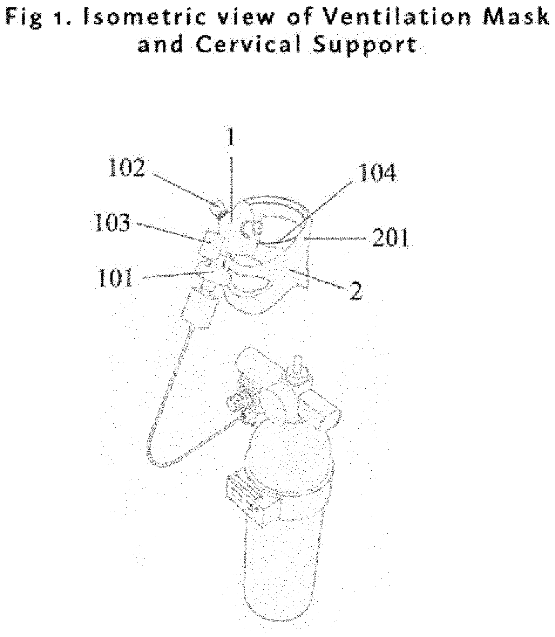

**FIG 1****.** Isometric view of the ventilation mask and the vertical support

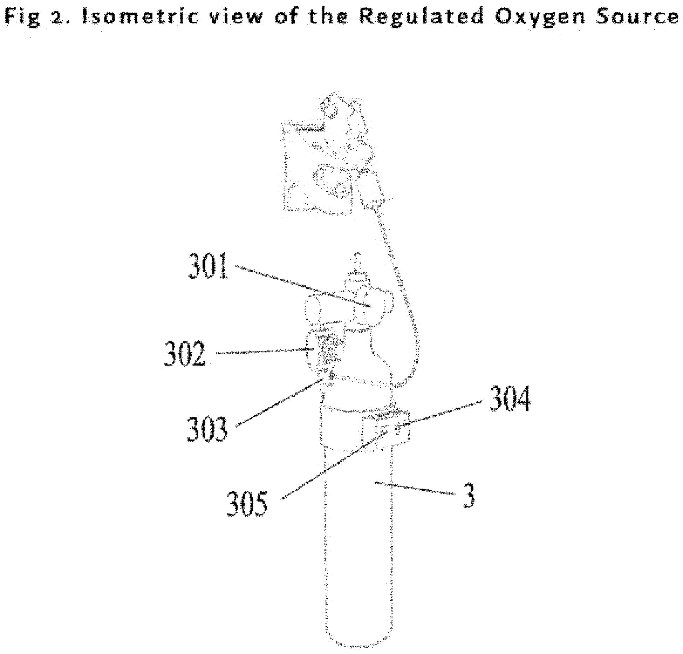

**FIG 2****.** Isometric view of the oxygen regulation source

### DETAILED DESCRIPTION OF THE INVENTION

An autonomous method and system for emergency respiratory failure reanimation. Pulmonary reanimation is done through a system comprised of a ventilation face mask which includes a CO2-detecting sensor, an auto-regulated oxygen source, and a cervical support piece. This system and method can be used by people with no medical emergency training, due to the system's autonomous response, as both the volume and frequency of the oxygen supply are software-calculated by entering the patient's age. The system also assists in the opening of the air passage through the use of the cervical support piece, allowing for optimum oxygen flow in respiratory failure situations. To develop the proposed action, the invention is equipped with a ventilation mask (1) that covers the nose and mouth, with a pressurized oxygen entry orifice (101) and a CO2 release vent (102) which opens by negative pressure and closes by positive pressure, that is to say, when oxygen enters it closes, and immediately after, it takes opposite action to allow for CO2 to exit. The ventilation mask (1) is characterized by a CO2 sensor (103) connected to software technology, which determines the patient's respiratory state by measuring their CO2 concentration, and subsequently releasing the correct volume and frequency of oxygen autonomously. The mask also has an anchorage device (104) on the cervical support (2) which maintains the support in a fixed position, adjacent to the face, which prevents oxygen from escaping via the patient's facial contour.

The Autonomous Emergency System for Respiratory Failure System and Method presented in this patent document has a cervical support (2) characterized by its anchorage device (201) which adheres to the ventilation mask (1); additionally, this support has been redesigned so that its structure is directly related to the positioning of the patient's head, to allow the opening of the airway and thus, assisting in a more efficient and effective pulmonary reanimation. The Autonomous Emergency System for Respiratory Failure also has an auto-regulated oxygen vent (3), which is connected to an energy source that powers the system, as well as a pressure source containing 3 valves: a pressure valve (301) a flow valve (302) as well as an opening valve (303), all connected to software-operated technology which allows for their oxygen release management. The oxygen source has two dials: an on/off dial (304) and one for the patient's age -adult or child-(305). The on/off dial (304) is regulated by the software program, which detects the presence or absence of CC2.

The Autonomous Emergency System for Respiratory Failure presented in this document is characterized by a defined structure of the phases necessary to perform pulmonary reanimation via oxygen supply, which is divided as follows:
- Locate the patient in a supine position, with the head resting on the cervical support, which has been designed and located to allow optimum airway management.
- Once the patient is identified as being unconscious or in a state of emergency, the provider must put the ventilation mask on the patient (1) securing it to the cervical support -via the anchorage device (201). At this point, the CO2 valve (102), located on the ventilation mask -which remains open by default, will immediately close as the oxygen flow begins. This is a cyclical process that occurs in 6 seconds, during which the first second supplies 500 ml of oxygen at a pressure of 50 cms of water, and then remains open for 5 seconds.
- The next step is to determine the patient's age -adult or child- and enter it via the dial (303), which is located on the auto-regulated oxygen source (3).
- The following step is to turn the system on via the on/off dial (302) which is located on the auto-regulated oxygen source (3).
- At this point, the system's software will set the rest of the resuscitation in motion based on the patient's age.
- The software will recognize once the patient has a stable breathing state and will shift to a maintenance oxygen supply. The machine will eventually turn off automatically.
- (Note: two types of masks will be implemented -one for adults, and another for children-considering their different physiognomy, anthropometry, and morphology. Sensor and electrical components will remain the same.)

### BIBLIOGRAPHY

**1.**US8827899B2 - Disposable endoscopic access device and portable display - Google Patents
**2.** https://patents.google.com/patent/US8414481B2/en?oq=US+8414481+B2
**3.** https://patents.google.com/patent/CN107348938A/en?oq=CN107348938A
**4.** https://patents.google.com/patent/CN209048096U/en?oq=CN209048096U
**5.** https://patents.google.com/patent/CN211130978U/zh?oq=CN211130978U

## Claims

1. An autonomous emergency method and system for respiratory failure and pulmonary reanimation via a ventilation mask equipped with a CO2 sensor, auto-regulated oxygen source, and cervical support, comprised of:
• Ventilation mask (1) that covers the nose and mouth, with a pressurized oxygen entry orifice (101), a CO2 release vent (102), a CO2 sensor (103) connected to software technology, and an anchorage device (104) which attaches to the cervical support (2).
• A cervical support (2), with an anchoring device (201) that attaches to the ventilation mask. The cervical support has been designed to have a direct relation with the positioning of the head.
• An auto-regulated oxygen vent (3), which is connected to an energy source, and is **characterized by** 3 valves: a pressure valve (301) a flow valve (302) as well as an opening valve (303), all connected to software-operated technology. The oxygen source has two dials: an on/off dial (304) and one for the patient's age -adult or child -(305).
• A method for performing pulmonary reanimation through the supply of an injection of oxygen to the patient's lungs.

2. An autonomous method and system for emergency respiratory failure reanimation. Pulmonary reanimation is done through a system comprised of a ventilation face mask which includes a CO2-detecting sensor, an auto-regulated oxygen source, and a cervical support piece. The CO2 escape valve (102) mentioned in Claim 1 opens by negative pressure and closes by positive pressure, that is to say, when oxygen enters it closes, and immediately after, it takes the opposite action to allow for CO2 to exit.

3. An autonomous method and system for emergency respiratory failure reanimation. Pulmonary reanimation is done through a system comprised of a ventilation face mask with a CO2-detecting sensor, an auto-regulated oxygen source, and a cervical support piece. The software technology mentioned in Claim 1 determines the patient's respiratory state by measuring the CO2 concentration, which initiates the required oxygen volume and frequency supply based on the patient's age. An autonomous method and system for emergency respiratory failure reanimation. Pulmonary reanimation is done through a system comprised of a ventilation face mask with a CO2-detecting sensor, an auto-regulated oxygen source, and a cervical support piece. The anchorage device (104) mentioned in Claim 1 adheres to the cervical support (2) and maintains the ventilation mask in a fixed position, adjacent to the face, which prevents oxygen from escaping via the patient's facial contour.

4. An autonomous method and system for emergency respiratory failure reanimation. Pulmonary reanimation is done through a system comprised of a ventilation face mask with a CO2-detecting sensor, an auto-regulated oxygen source, and a cervical support piece. The anchorage device (104) mentioned in Claim 1 has been designed to have a direct relation with the positioning of the head to allow for the opening of the air passage, and thus perform pulmonary reanimation.

5. An autonomous method and system for emergency respiratory failure reanimation. Pulmonary reanimation is done through a system comprised of a ventilation face mask with a CO2-detecting sensor, an auto-regulated oxygen source, and a cervical support piece. The pressure valves (301) mentioned in Claim 1 interact with the software and respond by opening and supplying oxygen based on its programming.

6. An autonomous method and system for emergency respiratory failure reanimation. Pulmonary reanimation is done through a system comprised of a ventilation face mask with a CO2-detecting sensor, an auto-regulated oxygen source, and a cervical support piece. The on/off dial mentioned in Claim 1 is regulated by the software technology depending on the presence or absence of C02.

7. An autonomous method and system for emergency respiratory failure reanimation. Pulmonary reanimation is done through a system comprised of a ventilation face mask with a CO2-detecting sensor, an auto-regulated oxygen source, and a cervical support piece. The method mentioned in Claim 1, is divided into the following stages:
• Locating the patient in a supine position, with the head resting on the cervical support, which has been designed and located to allow optimum airway management.
• Once the patient is identified as being unconscious or in a state of emergency, the provider must place the ventilation mask on the patient (1) securing it to the cervical support with the anchorage device (201). At this point, the CO2 valve (102), located on the ventilation mask -which remains open by default, will immediately close as the oxygen flow begins. This is a cyclical process that occurs in 6 seconds, during which the first second supplies 500 ml of oxygen at a pressure of 50 cms of water, and then remains open for 5 seconds.
• The following step is determining the patient's age -adult or child- and entering it via the dial (303), which is located on the auto-regulated oxygen source (3).
• The emergency provider must then turn the system on via the on/off dial (302) which is located on the auto-regulated oxygen source (3).
• At this point, the system's software will set the rest of the resuscitation in motion based on the patient's age.
• The software will recognize once the patient has a stable breathing state and will shift to a maintenance oxygen supply. The machine will eventually turn off automatically.
